# EUROPEAN PATENT SPECIFICATION

(11) **EP 3 213 695 B1**
(45) Date of publication and mention of the grant of the patent: **05.05.2021**
(21) Application number: 17162401.8
(22) Date of filing: 11.07.2013
(51) Int. Cl.: A61B 17/12, A61B 17/00

(54) **OCCLUSION DEVICE FOR AN ATRIAL APPENDAGE**
OKKLUSIONSVORRICHTUNG FÜR EIN HERZOHR
DISPOSITIF D'OCCLUSION D'UN APPENDICE AURICULAIRE

(30) Priority: 13.07.2012 US 201261671433 P
(43) Date of publication of application: 06.09.2017
(62) Divisional of application: 13740155.0
(73) Proprietor: Boston Scientific Scimed, Inc., Maple Grove, MN 55311 (US)
(72) Inventor: PEIFFER, Dennis A, Brooklyn Park, MN 55443 (US); TISCHLER, Brian Joseph, New Brighton, MN 55112 (US); LEY, Timothy J, Shoreview, MN 55126 (US); CLARK, Christopher J, St. Michael, MN 55376 (US); CHAU, Thyna M., Woodbury, MN 55125 (US)
(74) Representative: Vossius & Partner Patentanwälte Rechtsanwälte mbB

(56) References cited:
- US-A1- 2003 032 976
- US-A1- 2006 247 680
- US-A1- 2007 250 115
- US-A1- 2009 099 647
- US-A1- 2012 035 643

## Description

### Background of the Invention

The invention relates to occlusion devices and methods of manufacturing the same. More specifically, the invention relates to occlusion devices that prevent the dispersal of thrombi formed in an atrial appendage into the blood circulation system. In particular, the invention relates to devices having a cage-like structure that are adapted for implantation into the left atrial appendage and prevent blood clots formed therein from being released into the left atrium as well as methods for manufacturing such devices.

Structural heart disease or other cardiac conditions can result in atrial fibrillation, which in turn may cause blood to pool or stagnate in the patient's atrial appendage. Thrombi (i.e. blood clots) are prone to form in the atrial appendages with stagnant blood. The blood clots may subsequently break off and migrate to the brain leading to stroke, or to other parts of the body causing loss of circulation to the affected organ. The left atrial appendage (LAA), which is a pouch-like extension of the left atrium, happens to be a particularly likely site for harmful blood clot formation. Thromboembolic events such as strokes are frequently traced to blood clots from the LAA. Clinical studies show that the majority of blood clots in patients with atrial fibrillation are found in the LAA.

The risk of stroke in patients with atrial fibrillation may be reduced by drug therapy, for example, by using blood thinners such as Coumadin. However, not all patients can tolerate or handle the blood thinning drugs effectively. Alternative methods for reducing the risk of stroke involve surgery to remove or obliterate the LAA. Other proposed methods include using mechanical devices to occlude the atrial appendage opening and thereby stop or filter blood flow from the atrial appendage into its associated atrium.

A need exists for improved filtration or occlusions devices for use in the atrial appendage as well as for improved methods for manufacturing such devices.

US 2009/0099647 A1 discloses a vascular occlusion, flow restriction, shunt or filter device that comprises an assembly of at least two, of a number of selectable discrete interconnectable, interchangeable components. At least one component is of the type fabricated from metal strands braided into a tubular metal fabric having an expanded preset configuration and an elongated, collapsed reduced diameter configuration for delivery through a catheter to a treatment site, and the device is shaped to create an occlusion, flow restriction or shunt when placed in an opening in a body organ or vessel, the woven metal fabric having a memory property whereby the medical device tends to return to said expanded preset configuration when unconstrained.

### Summary of the Invention

The present invention is defined by an occlusion device and a method as recited in the independent claims. The dependent claims define embodiments.

The invention is directed, in at least one embodiment, to an occlusion device for an atrial appendage. The device has proximal and distal ends and a central axis and comprising a cage-like structure formed of struts. The struts have proximal strut ends and distal strut ends. At the proximal end of the device, the struts extend towards the central axis and are connected to each other at their proximal strut ends. At least some of the struts are connected to each other at their distal strut ends within the cage-like structure so that the struts form an atraumatic distal end of the device.

The cage-like structure may be cut from a unitary tubular body. It is also within the scope of the invention for the cage-like structure to be made from more than one body using cutting or other techniques.

The struts may have a substantially polygonal cross section. It is also within the scope of the invention to utilize struts with other shaped cross sections. The struts may form a plurality of closed polygonal cells having vertices where the struts merge into each other at said vertices. It is also within the scope of the invention for the struts to form other shape cells.

The atraumatic distal end of the device comprises inwardly bent struts. At least some of the ends of the bent struts point in a direction towards the proximal end of the cage-like structure. Typically, at least some of the struts are bent such that their distal strut ends extend substantially parallel to the central axis.

At least some of the distal strut ends may optionally provide an anchor. Typically, at least some of the struts providing the anchor extend through the cage-like structure.

Typically, at least some of the distal strut ends are connected to proximal strut ends.

The proximal strut ends may be connected to each other outside of the cage-like structure or within the cage-like structure. Optionally, the proximal strut ends may be connected to each other by a proximal collar formed integrally therewith.

The distal strut ends are connected to each other by a tube that may be crimped on and/or welded to the distal strut ends and/or a collar comprising several openings for receiving the distal strut ends. Welding, soldering, and adhesive may additionally be used.

The distal strut ends differ in part or completely in wall thickness and/or a strut width from the other struts of the cage-like structure.

The cage like structure may be formed of a single cut structure.

Optionally, the occlusion device may further comprise a filter.

The occlusion device further comprises a threaded insert at the proximal end.

According to the invention, there is provided an occlusion device having proximal and distal ends and a central axis and comprising a cage-like structure formed of struts. The struts have proximal strut ends and distal strut ends. At the proximal end of the device, the struts extend towards the central axis and are connected to each other at their proximal strut ends. At the distal end of the device, at least some of the struts extend toward the central axis and the proximal end, and are connected to each other at their distal strut ends such that the distal strut ends are located proximal to the distal most part of the device.

The invention is also directed to a method of manufacturing an occlusion device for an atrial appendage. The method comprises the steps of cutting a tubular body having proximal and distal ends to provide a tubular structure having struts, at least some of the struts at the distal end having loose distal strut ends, expanding at least part of the tubular structure; bending at least some of the loose distal strut ends towards the inside of said tubular structure such that the loose distal strut ends point in a direction towards the proximal end of the tubular structure, and connecting at least some of the loose distal strut ends to each other.

### Brief Description of the Drawings

The Figures described below disclose embodiments of the invention for illustrational purposes only. In particular, the disclosure provided by the Figures is not meant to limit the scope of protection conferred by the invention. The Figures are schematic drawings only and embodiments shown may be modified in many ways within the scope of the claims. In the context of the disclosure, like references numerals in the Figures refer to the same or corresponding features.
Figure 1 shows a side elevational view of an expanded occlusion device.
Figure 2 shows a cross-sectional view of the device illustrated in Figure 1.
Figure 3 shows a front elevational view of the device illustrated in Figure 1.
Figure 4 shows a side elevational view of an expanded occlusion device comprising a filter membrane.
Figure 5 shows a perspective view of the occlusion device illustrated in Figure 1 mounted on a tether wire.
Figure 6A shows a schematic sectional view illustrating the basic structure of an occlusion device wherein proximal strut ends are connected to each other within the cage-like structure
Figure 6B shows a schematic sectional view illustrating the basic structure of an occlusion device according to another embodiment of the invention wherein at least some of the distal strut ends provide an anchor.
Figure 6C shows a schematic sectional view illustrating the basic structure of an occlusion device wherein at least some of the distal strut ends are connected to the proximal strut ends.
Figure 7A shows a schematic sectional view illustrating the basic structure of an occlusion device wherein the distal strut ends are connected to each other by a collar comprising several openings for receiving the distal strut ends.
Figures 7B and 7C show schematic cross sectional and side elevational views of the collar illustrated in Figure 7A, respectively.
Figures 8A to 8D show different manufacturing stages of an occlusion device produced by a method according to the invention.
Figure 9 shows a pattern of a tubular structure that may be used for manufacturing an occlusion device according to the present invention in an unrolled and flattened state.
Figure 10 shows a strut with a barb extending therefrom.
Figure 11 shows a perspective view of an embodiment of an occlusion device.
Figure 12 shows another perspective view of the occlusion device of Figure 11.
Figure 13 shows a side view of the occlusion device of Figure 11.
Figure 14 shows an end view of the occlusion device of Figure 11.
Figure 15A shows an embodiment of an occlusion device with a tether wire having a threaded fixture extending therefrom.
Figure 15B shows the threaded fixture of Fig. 15A in greater detail.
Figure 16 shows a top view of a centering pin inserted into a tube along with the ends of the distal strut ends in order to arrange the strut ends around the inner wall of the tube.

### Detailed Description of the Drawings

In the context of the present disclosure, the terms "distal" and "proximal" are used according to their established meaning in the field of percutaneous endovascular devices. As such, the term "proximal" refers to those parts of the device which, when following a delivery catheter or delivery instrument during regular percutaneous delivery, are closer to an end of the catheter or instrument that is configured for manipulation by the user (e.g., a physician). In contrast, the term "distal" is used to refer to those parts of the device that are more distant from the end of the catheter or instrument that is configured for manipulation by the user and/or that are inserted further into the body of a patient. Accordingly, in a device for use in the atrial appendage the proximal end may face towards the atrium when the device is deployed in an auricle.

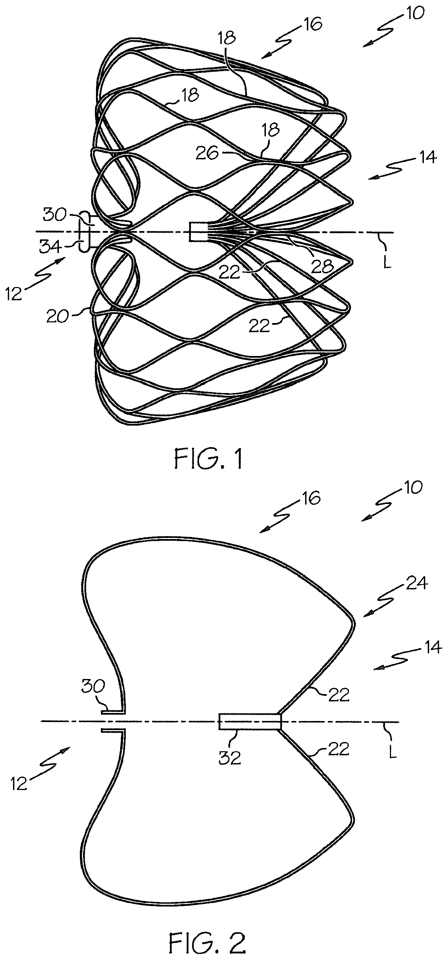

Figure 1 shows a side elevational view of an expanded occlusion device 10. As shown, the device 10 comprises a proximal end 12 and a distal end 14 as well as a central axis L and a cage-like structure 16 formed of struts 18. The struts 18 are formed from a cut structure so that they are integrally connected with each other. As such, the struts 18 may form generally polygonal cells with vertices 26 at which the struts 18 merge into each other. It is also within the scope of the invention for the struts to form cells of other shapes. The struts 18 may have a substantially polygonal cross section although struts with cross-sections having non-polygonal shapes may also be used.

The cage-like structure 16 forms a closed three dimensional frame, i.e., a frame closed on both ends 12, 14. The struts 18at the proximal end 12, i.e., proximal strut ends 20, which may be somewhat S-shaped, extend to the central axis L and are connected to each other. In case of the illustrative embodiment, the proximal strut ends 20 are connected at a proximal collar or hub 30. When the device is produced by cutting a tubular structure or a planar sheet, such proximal collar 30 may be provided by ending the cuts between the struts 18 at a sufficient distance from the proximal end 12 so as to define a collar between the ends of the cuts and the proximal end 12. Thus, at least some or all of the struts 18 forming the proximal strut ends 20 of the device 10 are attached at the collar 30. The proximal collar 30 may be provided with an insert 34 for attaching the device 10 to a device tether or shaft (e.g., a tether wire).

Moreover, as also illustrated in the sectional view of Figure 2, distal strut ends 22 may be connected to each other within the cage-like structure 16. At least some of the distal strut ends 22 are bent inwardly so as to point in a direction towards the proximal end 12 of the cage-like structure 16. In the illustrated embodiment, the distal-most part of the distal strut ends 22 extend substantially parallel to the central axis L. The bent distal strut ends 22 thus form an atraumatic distal end 24 of the device 10. The struts 18 may be bent such that the distal end 14 of the device is atraumatic, preferably in both the constrained and the deployed state of the device.

As further shown in Figures 1 and 2, the cage-like structure 16 may have a tapered shape. For example, at least a segment of the cage-like structure 16 may taper towards the distal end 14. In some embodiments, the device may have a generally cone-like, for example, frusto-conical, or cylindrical shape. Such shapes may allow the device 10 to accommodate more closely to the natural shape of the LAA while exerting a tolerable outward contact pressure against the walls of the atrial appendage in order to provide an interference-like fit and hold the device 10 in place. The outward contact pressure may result from the designed springiness or elasticity of the cage-like structure.

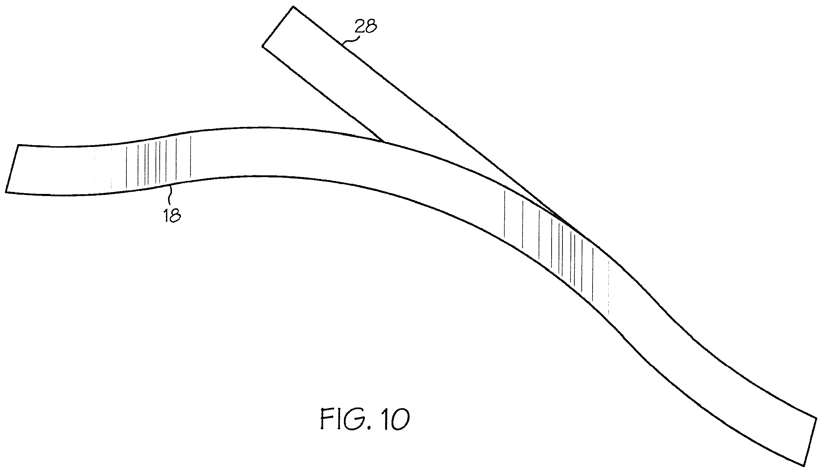

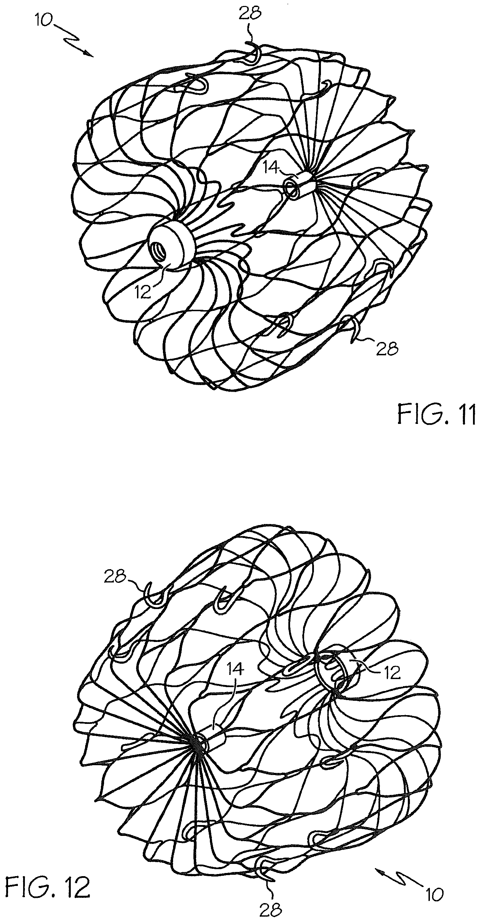

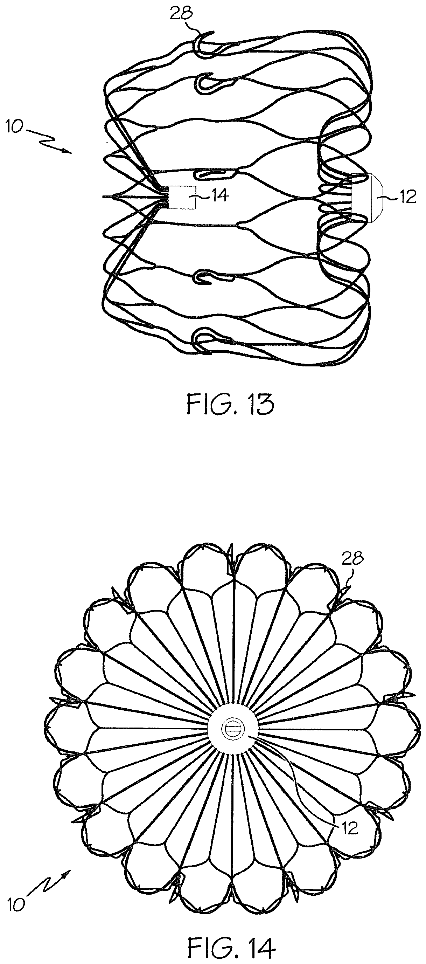

In order to stabilize the position of the device 10 following implantation, the device can also comprise one or more anchors, which may have any suitable form. As illustrated in Figures 1 and 10, the anchor may be pins or barbs 28 adapted for engaging the wall of the atrial appendage. The barbs 28 may extend from the struts 18 delimiting an outer perimeter of the cage-like structure 16. The barbs 28 can be formed integrally with the struts 18, e.g., by laser cutting. Barbs 28 may also be seen in the expanded occlusion device 10 of Figures 11-14. The device may have as many as 6, 12, 18, 26 or any other suitable number of anchors.

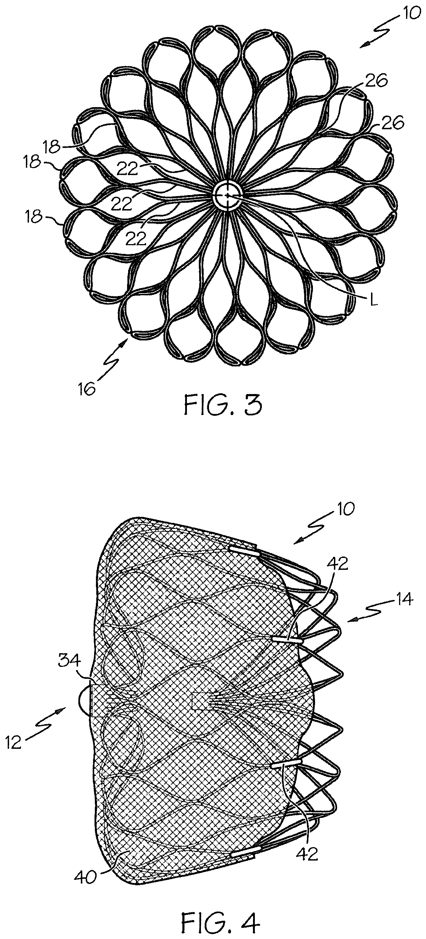

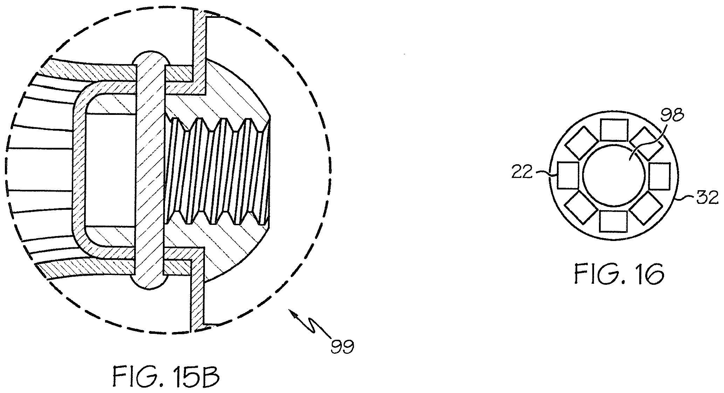

As further shown in Figure 2, the distal strut ends 22 are connected to each other by a tube 32 that is crimped to the distal strut ends 22. Alternatively or additionally, the tube 32 may be fixed to the distal strut ends 22 by welding, soldering or adhesives. The strut ends may be secured to the tube at either end of the tube. The struts may extend the entire length of the tube or only part of the length of the tube. As shown in Figure 16, a centering pin 98 may be inserted into the tube 32 along with the ends of the distal strut ends 22 in order to arrange the ends around the inner wall of the tube 32. In some embodiments, at least six, more typically at least ten, even more typically at least twelve or more struts 22 may be connected within the cage-like structure 16 to form the distal end 14 of the device. Figure 3, which depicts a top elevational view of the device 10 illustrated in Figure 1, for example, shows eighteen distal strut ends 22 connected within the cage-like structure 16.

Figure 4 shows a side elevational view of an expanded occlusion device 10. The device 10 includes a filter comprising a filter membrane 40 supported on the outer surface of the cage-like structure 16. More specifically, the filter membrane 40 is affixed at the proximal end 12 of the device. It should be noted, however, that, alternatively or additionally, a filter membrane may be provided at the distal end 14. Furthermore, the filter membrane(s) 40 may be provided along the outside of the cage-like structure 16 or therein.

The filter membrane may be attached to the cage like structure 16 by any suitable technique, including hooks or barbs provided at the cage-like structure 16 and/or, as in the exemplary embodiment illustrated in Figure 4, one or several filaments 42. Filaments 42 may be threaded through holes in the filter membrane 40 and tied to the struts 18 in order to secure the filter membrane 40 to the cage-like structure 16.

As mentioned above, the filter membrane may be made of a blood-permeable material having fluid conductive holes or channels extending across the membrane. The filter membrane may be fabricated from any suitable biocompatible material. These materials include, for example, ePFTE (e.g., Gore-Tex®), polyester (e.g., Dacron®), PTFE (e.g., Teflon®), silicone, urethane, metal fibers, and other biocompatible polymers. The hole sizes in the blood-permeable material may be chosen to be sufficiently small so that harmful-size emboli are filtered out from the blood flow between the appendage and the atrium. Suitable hole sizes may range, for example, from about 50 to about 400 microns in diameter. In embodiments, the filter membrane may be made of polyester (e.g., Dacron®) weave or knit having a nominal hole size of about 125 microns. The open area of the filter membrane (i.e., the hole density) may be selected or tailored to provide adequate flow conductivity for emboli-free blood to pass through the atrial appendage ostium. Further, portions of filter membrane may be coated or covered with an anticoagulant, such as heparin or another compound, or otherwise treated so that the treated portions acquire antithrombogenic properties to inhibit the formation of hole-clogging blood clots.

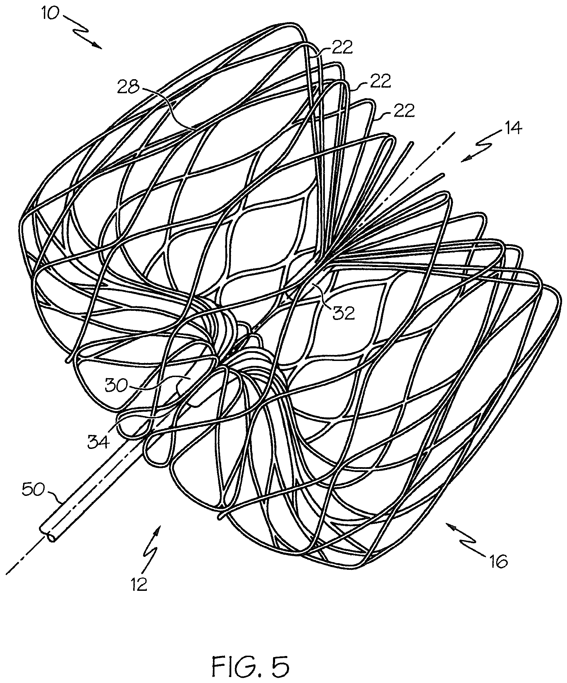

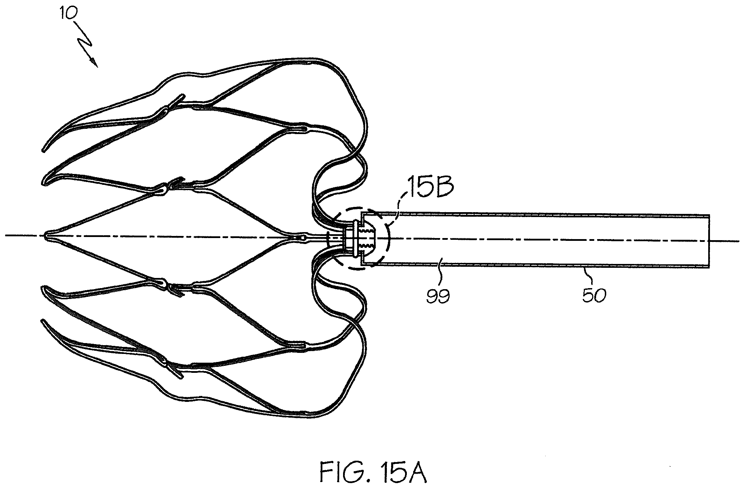

Figure 5 depicts a perspective view of the device 10 illustrated in Figure 1. As shown therein, the insert 34 has a threaded socket A tether wire 50 having a threaded fixture for engaging the insert 34 may be threaded into the socket in order to manipulate the device 10. The threaded socket may be suitable for rotatably engaging and/or releasing the occlusion device 10. An embodiment of an occlusion device 10 with a tether wire 50 having a threaded fixture 99 is also shown at 10 in Figure 15A and Figure 15B. It should be noted that, additionally or alternatively, any other suitable attachment may be provided.

The device 10 shown in Figures 1 to 5 is a self-expanding device and is shown in its natural unconstrained expanded state. The cage-like structure 16 of the device 10 may be fabricated in different-sizes as necessary or appropriate for use in different sizes of atrial appendages. The illustrated structure may be, for example, about 25.4 mm (about one inch) in diameter and about 25.4 mm (about one inch) long in its natural expanded state. For delivery (e.g., percutaneous delivery), the device 10 may be compressed to a narrow diameter tubular shape and fitted into a narrow diameter catheter or delivery sheath. Preferably, the device may be compressed to a diameter of less than 4 mm, more preferably of less than 3 mm and recover to its natural shape subsequently upon release from the sheath. For applications in small vessels, the device may be compressed to a diameter of less 2 mm or less while for larger diameter vessels such as the aortic valve, the device may be compressed to a diameter of less than 5 mm.

The struts 18 of the cage-like structure 16 may be made of any suitable elastic material, for example, nitinol or spring steel. In the case of shape memory materials such as nitinol, the device may be provided with a memorized shape and then deformed to a reduced diameter shape. The device may restore itself to its memorized shape upon being heated to a transition temperature and/or having any restraints removed therefrom.

Depending on the specific embodiments and the requirements for the intended use, the device may also be made from any other suitable biocompatible material including one or more polymers, one or more metals or combinations of polymer(s) and metal(s). Examples of suitable materials include biodegradable materials that are also biocompatible. In this context, the term "biodegradable" is used to denominate a material that undergoes breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and/or alloys of any of the above-mentioned metals. Examples of suitable alloys may include platinum-iridium alloys, cobalt-chromium alloys (e.g., Elgiloy and Phynox, MP35N), nickel-titanium alloys and nickel-titanium-platinum alloys.

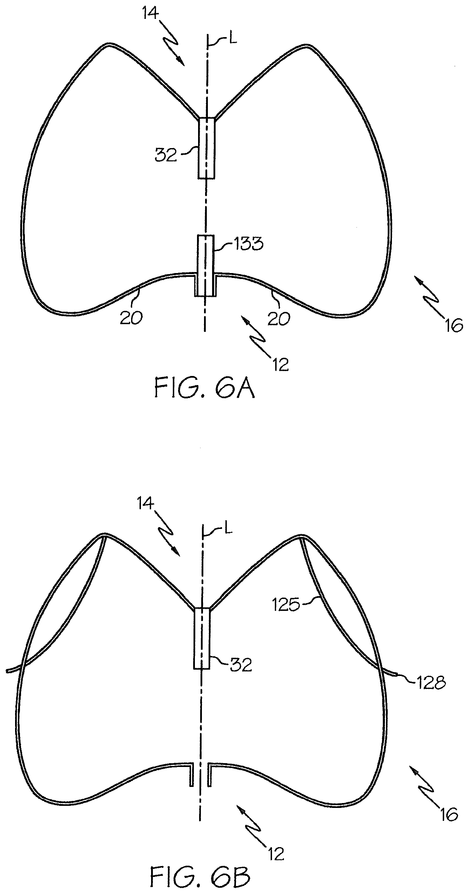

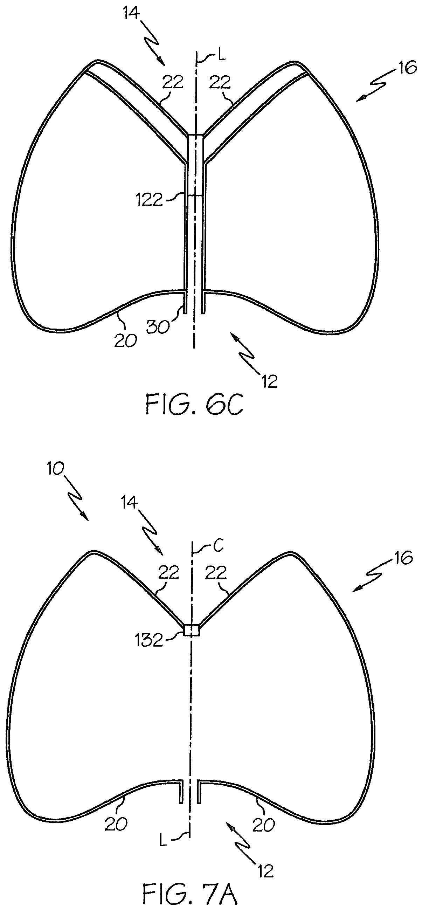

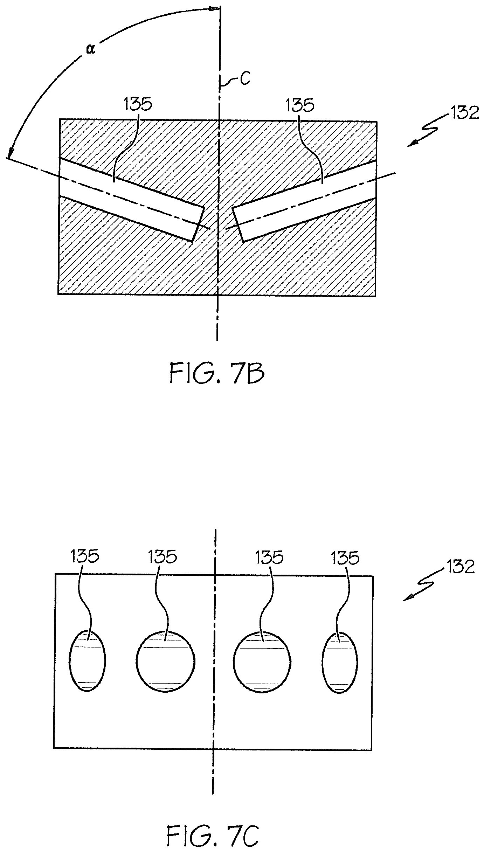

Figures 6A to 6C, 7A and 7B illustrate further optional features that may be provided in conjunction with the device 10 of Figures 1 to 5. In order to avoid repetitions, only those features differing from the device described above will be addressed. Like reference numbers denominate the same or corresponding features.

Figure 6A shows a schematic sectional view illustrating an embodiment of the cage-like structure 16 for devices 10. As shown therein, at least some or all of the proximal strut ends 20 may be connected within the cage like-structure 16. For example, separate struts may be formed at the proximal end of the tubular structure when cutting it, which may then be bent towards the inside of the cage-like structure 16 and connected therein.

The proximal strut ends 20 may be connected to each other by a tube 133 that is crimped on, soldered, adhered and/or welded to the proximal strut ends 20. A centering pin may be used in this context to arrange the proximal strut ends 20 evenly at the inner wall of the tube 133. According to other embodiments, a collar comprising several openings for receiving the proximal strut ends 20 may be employed. The proximal strut ends 20 may also be welded, soldered and/or adhered to each other directly.

According to embodiments of the invention schematically illustrated in Figure 6B, at least some of the distal strut ends 22 may provide an anchor 128. As shown, the distal strut ends 22 providing the anchor 128 extend through the cage-like structure 16, for example, from the inside of the cage-like structure 16 towards the outside. As illustrated, the anchor 128 may be provided in a central or distal part of the cage-like structure 16, for example, within the distal half or the most distal third thereof relative to the entire length of the cage-like structure 16 along the central axis L. It should be noted that such anchor(s) are optional. They may be provided alternatively or additionally to the anchor 28 described above.

Figure 6C depicts an embodiment of the cage-like structure 16 wherein some of the distal strut ends (i.e., distal strut ends 122) extend partially along the central axis L through the cage-like structure 16 towards the proximal end 12 of the device 10 to the proximal strut ends 20. The distal strut ends 122 may be connected to the proximal strut ends 20, for example, at a location where the proximal strut ends 20 are connected to each other (e.g., at the proximal collar 30). In certain embodiments, some or all of the distal strut ends 22 may extend through the cage like structure 16.

Figure 7A shows a schematic sectional view illustrating the cage-like structure of another occlusion 10 device. In this embodiment, the distal strut ends 22 are connected by a distal collar 132 comprising several openings 135 (see Figure 7c) for receiving the distal strut ends 22. As further shown in Figures 7B and 7C, which depict schematic cross sectional and side elevational views of the distal collar 132, the openings 135 provided around the circumference of the distal collar 132 may extend at an angle α with respect to the central axis C of the collar 132. Axis C may be concentric with the central axis L of the device 10. The angle α may be between 0° and 70°. The distal collar 132 may be substantially cylindrical and/or may be provided with, for example, six, ten, twelve or eighteen or more openings 135, corresponding to the number of distal strut ends 22 to be attached to the collar. In some embodiments, a similar structure may be used to connect at least some of the proximal strut ends 20.

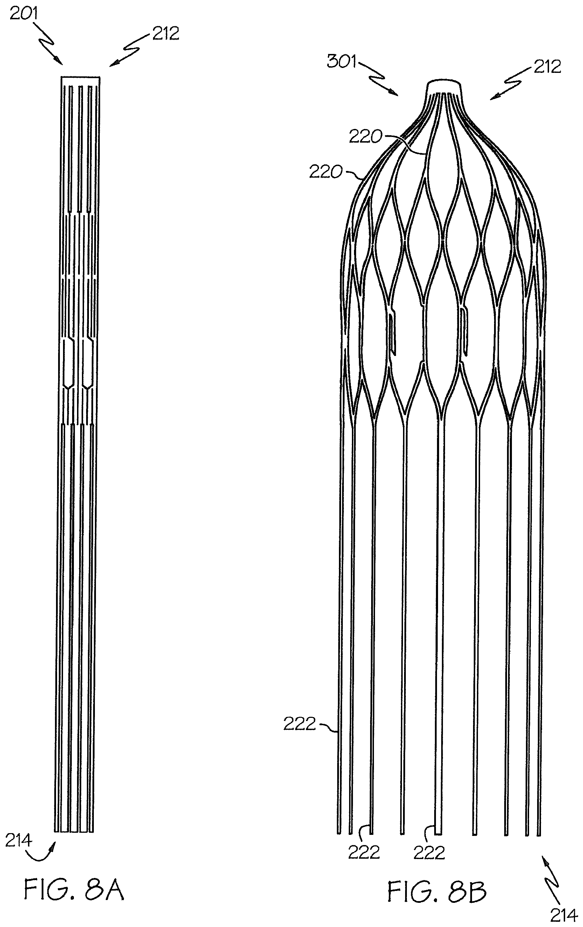

Figures 8A to 8D show different stages of a method for manufacturing an occlusion device 10 according to the invention. Figure 8A illustrates a tubular structure 201 having a proximal end 212 and a distal end 214 and comprising a plurality of struts. The struts form loose distal strut ends 222 at the distal end of the device 214. The tubular structure 201 is produced by cutting a tubular nitinol body (not shown).

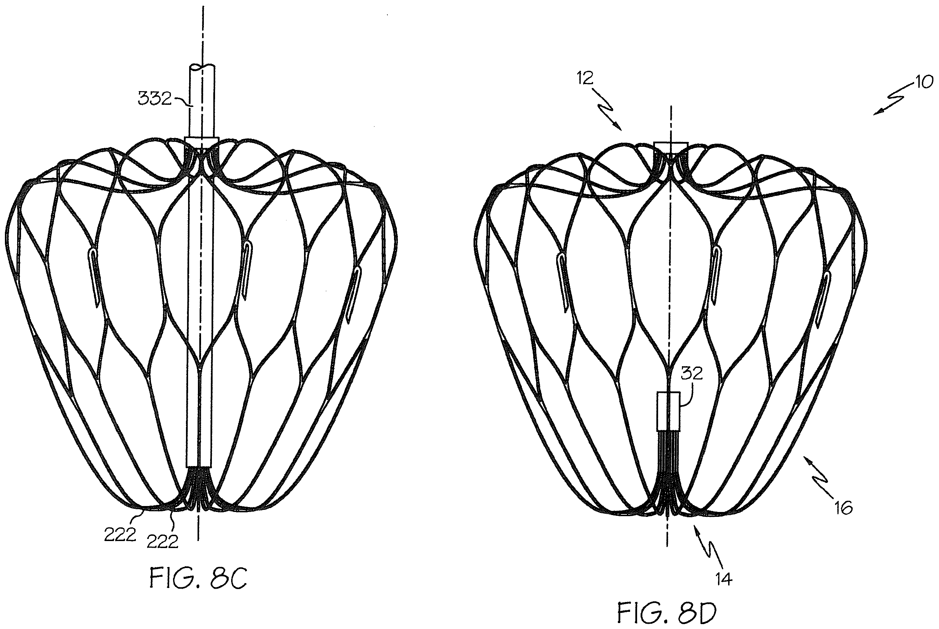

As illustrated in Figure 8B, the tubular structure may subsequently be heat treated and expanded by means of a mandrel (not shown) in order to provide a preform 301. A forming tool (not shown) may be used to provide the proximal strut ends 220 of the preform 301 with a desired shape, for example, the S-shape illustrated in Figure 8C. The proximal strut ends 220 are connected to each other at the proximal end 212.

The loose distal strut ends 222 are then bent such that they have a directional component towards the proximal end 212. As shown in Figure 8C, the distal strut ends 222 may be bent towards the inside of the preform 301, such that the loose distal strut ends 222 point in a direction towards the proximal end 212 of the preform 301. A tube 332 (e.g., a hypotube) is introduced through the proximal end 212 (e.g., through a proximal collar 230) and the loose distal strut ends 222 are inserted into the tube 332. The hypotube 332 is then crimped and/or welded to the distal strut ends 222 in order to connect the ends to each other in a fixed manner. As further illustrated, the hypotube 332 is then cut and pulled back through the proximal end 212. The remaining crimped portion 32 holds the loose distal strut ends 222 together in a fixed and secure manner. Accordingly, the cage-like structure 16 with closed proximal and distal ends 12, 14 is formed (see Figure 8D). In other embodiments, the loose distal strut ends 222 may be connected to each other by welding, soldering and/or by use of an adhesive. The tubular structure may be microblasted and/or electropolished.

Further steps may be performed, inter alia, to provide cage-like structures according to the embodiments shown in Figures 6A to 6C and 7A.

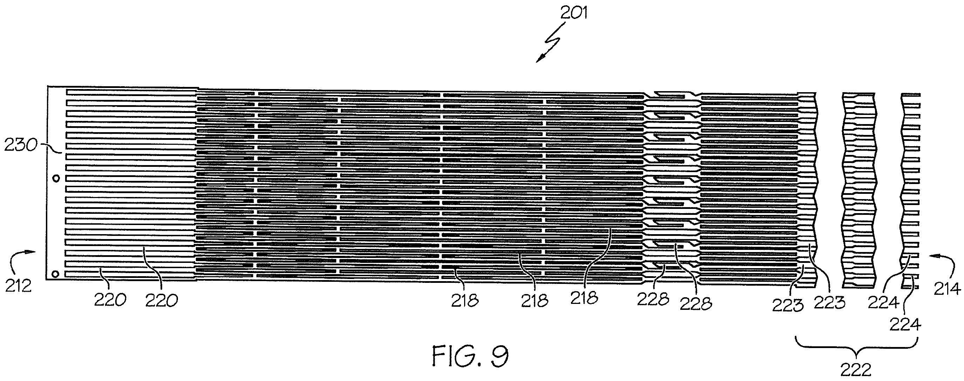

Figure 9 shows a tubular structure 201 that may be used for manufacturing an occlusion device 10 according to the present invention. The structure is illustrated in an unrolled and flattened state in order to depict the pattern formed by the struts. The tubular structure 201 is produced by laser cutting a tubular body or other suitable processes such as rolling an etched and/or cut sheet of material.

As mentioned above, the tubular structure 201 comprises struts 218 that may be adapted and configured to form the struts 18 of the device 10 illustrated in Figures 1 to 5.

The struts 218 extend from a proximal end 212 of the tubular structure 201 to a distal end 214 of said tubular structure 201. At the proximal end 212, the proximal ends 220 of the struts 218 are configured and adapted to form the proximal end 12 of an occlusion device 10. As shown, the cuts between the proximal strut ends 220 of the struts 218 end at a distance from the proximal end 212 in order to leave a proximal collar 230, which integrally connects the proximal strut ends 220.

At the distal end 214, the tubular structure 201 comprises distal strut ends 222. The distal strut ends 222 may terminate in loose ends at or proximate the distal end 214, which are indicated at 224 in Figure 9. It should be noted in this context that Figure 9 provides a schematic representation and does not show the entire length of distal strut ends 222, which may, optionally, account for approximately 20% to 65%, preferably approximately 30% to 55%, more preferably approximately 40% to 50%, and most preferably approximately 45% of the length of the tubular structure. As mentioned above with respect to the device 10 shown in Figures 1 to 5 , the tubular structure 201 may comprise at least 6, at least 10, at least 12, or 18 or more loose distal strut ends.

The struts forming the loose distal strut ends 222 differ in wall thickness and/or strut width along their entire length or a section thereof. As such, the distal strut ends 222, for example, may have a first section 223 that is wider than a second section 224 (see Figure 9). In other embodiments, a middle or a distal end section of distal strut ends 222 may be provided with a larger or smaller wall thickness and/or strut width. Varying the wall thickness and/or the strut width may allow configuring the bending properties of the distal strut ends 222, thereby determining, inter alia, the shape of the distal end 12 of device 10 as well as its radial stability.

Anchoring struts 228 are optional and may be configured and adapted to provide the anchor 28 described above. The anchoring struts 228 may be formed when providing the tubular structure and may be integrally connected to struts 218.

The invention, in one or more embodiments, is directed to an occlusion device having proximal and distal ends and a central axis and comprising a cage-like structure formed of struts. The struts have proximal strut ends and distal strut ends. At the proximal end of the device, the struts extend towards the central axis and are connected to each other at their proximal strut ends. At the distal end of the device, at least some of the struts are invaginated inward toward the central axis and the proximal end, and are connected to each other at their distal strut ends such that the distal strut ends are located proximal to the distal most part of the device.

The invention is directed, in one or more embodiments, to an occlusion device having proximal and distal ends and a central axis and comprising a cage-like structure formed of struts. The struts have proximal strut ends and distal strut ends. At the proximal end of the device, the struts extend towards the central axis and are connected to each other at their proximal strut ends. At the distal end of the device, at least some of the struts extend toward the central axis and the proximal end, and are connected to each other at their distal strut ends such that the distal strut ends are located proximal to the distal most part of the device.

The invention relates to an occlusion device for use in an atrial appendage of a patient. The device may filter or otherwise modify or even block blood flow between an atrial appendage and the associated atrium. The device may be configured and adapted for deployment into an atrial appendage, i.e., the LAA. However, it will be understood that the device can also be placed across other apertures in the body, e.g., apertures through which blood flows. The device may also be adapted for use for RF based ablation.

The device may have a proximal end and a distal end as well as a central axis and a cage-like structure formed of struts. The struts each have a proximal strut end and a distal strut end. At the proximal end of the device the struts extend towards the central axis and are connected to each other at their proximal strut ends. Further, at least some of the struts are connected to each other at their distal strut ends within the cage-like structure so that the struts form an atraumatic distal end of the device.

The device may be self-expanding, i.e., it may form an elastic structure that expands from a compressed state to a predetermined expanded state when being unconstrained. In a compressed state, the device may take a narrow diameter tubular shape that is convenient for fitting the device into a narrow diameter catheter or delivery tube for percutaneous delivery. The cage-like structure typically forms a mesh or frame that is closed at both ends and surrounds a three dimensional space when the device is in an expanded state. Alternatively or additionally, the device may be designed to be expandable by means of an expansion mechanism for expanding the device in situ, for example, an inflatable balloon.

The atraumatic distal end of the device according to the invention is atraumatic at least in its constrained delivery state, and more typically both in the constrained and a deployed state. As such, the atraumatic distal end may be configured to enhance structural compatibility of the device with the atrial appendage during deployment as well as after implantation of the device. This could, desirably, reduce the risk of perforation. Also, it could allow for one or more recaptures of the device in the catheter and for full recapturability of the device by the catheter while having a lower likelihood for strut entanglement.

The occlusion devices of the invention may be formed in several ways. In accordance with one embodiment the device is cut from a tubular body so as to provide the plurality of struts. The cuts may be formed in the tubular body, for example, by laser cutting, etching or other cutting techniques know in the art, particularly in the art of stent manufacturing. The struts of the device forming the cage-like structure may have a substantially polygonal cross-section or a cross-section of other, non-polygonal, shapes.

The device, in some embodiments of the invention, may also be characterized in that the struts form a plurality of closed polygonal cells having vertices, the struts merging into each other at said vertices. In other embodiments, non-polygonal cells may be provided. According to the invention, the cage-like structure may be formed from a single cut structure, e.g., a single tubular body. In this way all struts are integrally connected with each other so that the cage-like structure represents a unitary body.

In accordance with embodiments of the invention, the atraumatic distal end of the device comprises inwardly bent struts. In such a design, at least some of the struts at the distal end are bent towards the inside of the cage-like structure. As such, at least some, most or all of the ends or tips of the bent struts may be located inside the cage-like structure when the device is constrained and/or when the device is deployed.

According to at least some of the embodiments with bent struts, at least some of the ends of the bent struts point in a direction towards the proximal end of the cage-like structure. Preferably, at least some, most or all of the struts are bent such that their distal strut ends extend substantially parallel to the central axis. As discovered by the inventors, such architecture may provide the device with a combination of performance characteristics that are normally difficult to obtain. Inter alia, the device may be constrained to a low profile and have a high radial strength, which may effectively ensure expansion upon deployment and prevent collapse after implantation.

The cage-like structure or frame of the device according to the invention may be fabricated in different sizes, as necessary or appropriate for use in different sizes of atrial appendages or other suitable areas of the body. An exemplary cage-like structure may be about 25.4 mm (one inch) in diameter and about 25.4 mm (one inch) long in its natural expanded state. In the constrained state, it may be about 4 mm in diameter and 35 mm in length.

In some embodiments, the cage-like structure may have a tapered shape. For example, the device may be tapered towards the distal end such that an outer diameter proximate the proximal end of the device is larger than an outer diameter proximate the distal end of the device. Thus, the device may have a generally conical, preferably frusto-conical shape. Other shapes, e.g., a generally cylindrical shape, are also feasible.

When the device is expanded in an atrial appendage, it may be held in position by an outwardly directed contact pressure that the cage-like structure exerts against the walls of said atrial appendage, providing an interference-like fit of the device. The contact pressure may result from the designed springiness or elasticity of the cage-like structure or may be the result of plastic deformation.

Alternatively or additionally, the device may comprise one or more anchors, which may engage the wall of the atrial appendage in order to ensure long-term stability in the implanted position. Examples of such tissue-engaging anchors may be hooks, pins, barbs, wires with an atraumatic bulb, tips or other suitable structures. The anchor(s) may be in the form of stubs or barbs extending from the struts forming the cage-like structure and may be formed integrally therewith. For example, the anchor(s) may extend from struts delimiting the outer diameter of the cage-like structure. Alternatively or additionally, at least some of the distal strut ends may provide one or more anchors. In some embodiments of the invention, at least some of the struts providing the anchor(s) may extend from the interior through the cage-like structure outwardly. For example, at least some of the distal strut ends may extend from the inside of the cage-like structure towards the outside in order to provide the anchor(s). The anchor(s) may be provided in the central of distal part of the cage-like structure, for example, within the distal half or the most distal third of the device compared to the overall length from the proximal end to the distal end along the central axis. It should be noted that the anchor(s) are optional and may or may not be provided in the inventive devices according to the specific requirements determined by the intended use.

The proximal strut ends may be connected to each other by one or a combination of: a tube that is crimped on and/or welded to the struts, a collar comprising several openings for receiving the ends of the struts, welding, soldering, use of adhesive, etc. The distal strut ends are connected to each other by a tube into which the distal strut ends are inserted and/or a collar comprising several openings for receiving the distal strut ends. When the struts are connected by means of a tube, a centering pin may be used to arrange the struts at the inner wall of the tube. The struts may also be arranged at the inner wall of the tube via the use of a shrink tube or by attachment with filament such as wire.

Alternatively, the proximal strut ends may be integrally connected with each other. More specifically, the struts at the proximal end may remain connected to each other by a proximal collar or hub formed integrally therewith. When the device is produced by cutting a tubular structure, the cuts between struts forming the proximal end of the device may be ended at a sufficient distance from the proximal end of the tubular structure in order to leave a proximal collar or hub to which at least some or all of the proximal strut ends forming the proximal end of the device are attached.

In some embodiments, at least some or all of the proximal strut ends may be connected to each other within the cage-like structure. For example, separated proximal strut ends may be formed at the proximal end of the tubular structure when cutting it, which may then be bent towards the inside of the cage like structure and connected to each other therein. In one embodiment, some of the proximal strut ends are connected to each other outside the cage-like structure, for example by a proximal collar, while others are connected to each other within said cage-like structure. Accordingly, some of the proximal strut ends may be generally S-shaped while others may be generally C-shaped.

In another embodiment of the invention, at least some struts may extend from the distal end through the cage-like structure to the proximal end of the device, for example, along the central axis. In such an embodiment, the distal strut ends may be connected to the proximal strut ends, for example, where the proximal strut ends at the proximal end of the device are connected to each other. Such architectures may enhance stability of the device.

The struts forming the distal end of the device differ in wall thickness, strut width or both from the other struts of the cage-like structure. For example, the wall thickness and/or the width of the struts forming the distal end may be smaller or larger than the wall thickness and/or the width of other struts of the cage-like structure. Alternatively or additionally, a segment of the struts forming the distal end may have a different (e.g., smaller/larger) wall thickness and/or width. The segment may be provided at any suitable location along the struts, for example, at a proximal, a middle or a distal end section thereof. The wall thickness and/or the strut width of the struts that form the distal end of the device may be varied in order to define the bending properties (e.g., the curvature and/or the radial strength) of the struts forming the distal end of the device. In some embodiments, also the wall thickness and/or the strut width of other struts forming the cage-like structure may be varied, for example, along their entire length or a segment thereof.

In accordance with the invention, the device is provided with an insert that may be configured for attaching the device to a tether or shaft (e.g., tether wire). For this purpose, the insert may have, for example, a threaded socket so that a tether wire can be releasably attached from a proximal direction. However, other attachment means are likewise feasible and will be apparent to those skilled in the art.

The occlusion device according to the invention may additionally comprise a filter, for example, a filter membrane. The filter may be disposed along at least a portion of the cage-like structure, for example, along an outer or an inner segment thereof. For example, the filter membrane may cover a proximal portion of the cage-like structure (e.g., a proximal "hemisphere" or end thereof). In some embodiments, the filter membrane may span over the atrial facing surface of the device. Additionally or alternatively, the filter may be arranged at the distal portion of the device.

The filter can be attached by any suitable technique. For example, the filter may be supported by hooks or barbs extending from the cage-like structure. Alternatively or additionally, filaments may be used to tie the filter to the cells (e.g., at the vertices). At the proximal end, the filter membrane may be held between struts forming said proximal end and the insert and/or between the proximal collar and the insert.

The filter membrane may be made of a blood-permeable material having fluid conductive holes or channels extending across the membrane. The filter membrane may be fabricated from any suitable biocompatible material. These materials include, for example, ePFTE (e.g., Gore-Tex®), polyester (e.g., Dacron®), PTFE (e.g., Teflon®), silicone, urethane, metal fibers, and other biocompatible polymers. The sizes of the holes in the blood-permeable material may be chosen to be sufficiently small so that harmful-size emboli are filtered out from the blood flow between the appendage and the atrium. Suitable hole sizes may range, for example, from about 50 to about 400 microns in diameter. In embodiments, the filter membrane may be made of polyester (e.g., Dacron®) weave or knit having a nominal hole size of about 125 microns. The open area of the filter membrane (i.e., the hole density) may be selected or tailored to provide adequate flow conductivity for emboli-free blood to pass through the atrial appendage ostium. Further, portions of filter membrane may be coated or covered with an anticoagulant, such as heparin or another compound, or otherwise treated so that the treated portions acquire antithrombogenic properties to inhibit the formation of hole-clogging blood clots. The filter membrane assists in the occlusion of the atrial appendage. In particular, over time, the blood-clots captured by the filter, may lead to occlusion of the ostium of the atrial appendage.

The struts forming the cage-like structure may be made of any suitable elastic material, for example, nitinol or spring steel. Also shape memory materials may be used (e.g., nitinol). In this case, the device may be provided with a memorized shape and then deformed to a reduced diameter shape. The device may restore itself to its memorized shape upon being heated to a transition temperature and having any restraints removed therefrom.

Depending on the specific embodiments and the requirements for the intended use, the device of the invention may also be made from any other suitable biocompatible material including one or more polymers, one or more metals or combinations of polymer(s) and metal(s). Examples of suitable materials include biodegradable materials that are also biocompatible. A "biodegradable" material means that the material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, copolymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and alloys of any of the above-mentioned metals. Examples of suitable alloys may include platinum-iridium alloys, cobalt-chromium alloys including Elgiloy and Phynox, MP35N alloy, nickel-titanium alloys and nickel-titanium-platinum alloys.

The device of the invention may be provided with a one or more therapeutic agents, whether in coating form or otherwise. As used herein, the terms, "therapeutic agent", "drug", "pharmaceutically active agent", "pharmaceutically active material", "beneficial agent", "bioactive agent", and other related terms may be used interchangeably herein and include genetic therapeutic agents, non-genetic therapeutic agents and cells. A drug may be used singly or in combination with other drugs. Drugs include genetic materials, non-genetic materials, and cells.

A therapeutic agent may be a drug or other pharmaceutical product such as non-genetic agents, genetic agents, cellular material, etc. Some examples of suitable non-genetic therapeutic agents include but are not limited to: antithrombogenic agents such as heparin, heparin derivatives, vascular cell growth promoters, growth factor inhibitors, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof.

Other active agents include, but are not limited to, antineoplastic, antiproliferative, antimitotic, antiinflammatory, antiplatelet, anticoagulant, antifibrin, antiproliferative, antibiotic, antioxidant, and antiallergic substances as well as combinations thereof.

Examples of antineoplastic/antiproliferative/antimitotic agents include, but are not limited to, paclitaxel (e.g., TAXOL.RTM. by Bristol-Myers Squibb Co., Stamford, Conn.), the olimus family of drugs including sirolimus (rapamycin), biolimus (derivative of sirolimus), everolimus (derivative of sirolimus), zotarolimus (derivative of sirolimus) and tacrolimus, methotrexate, azathiprine, vincristine, vinblastine, 5-fluorouracil, doxorubicin hydrochloride, mitomycin, cisplatin, vinblastine, vincristine, epothilones, endostatin, angiostatin and thymidine kinase inhibitors.

While the preventative and treatment properties of the foregoing therapeutic substances or agents are well-known to those of ordinary skill in the art, the substances or agents are provided by way of example and are not meant to be limiting. Other therapeutic substances are equally applicable for use with the disclosed methods and compositions. See U.S. Patent Application Nos. 2010/0087783, 2010/0069838, 2008/0071358 and 2008/0071350. See also U.S. Patent Application Nos. 2004/0215169, 2009/0098176, 20120095396 and US 2009/0028785.

Derivatives of many of the above mentioned compounds also exist which are employed as therapeutic agents and of course mixtures of therapeutic agents may also be employed.

For application, the therapeutic agent can be dissolved in a solvent or a cosolvent blend, and an excipient may also be added to a coating composition.

Suitable solvents include, but are not limited to, dimethyl formamide (DMF), butyl acetate, ethyl acetate, tetrahydrofuran (THF), dichloromethane (DCM), acetone, acetonitrile, dimethyl sulfoxide (DMSO), butyl acetate, etc.

Suitable excipients include, but are not limited to, acetyl tri-n-butyl citrate (ATBC), acetyl triethyl citrate (ATEC), dimethyl tartarate (D, L, DL), diethyl tartarate (D, L, DL), dibutyl tartarate (D, L, DL), mono-, di- and tri-glycerol such as glycerol triacetate (triacetin), glycerol tributyrate (tributyrin), glycerol tricaprylate (tricarprin), sucrose octa acetate, glucose penta acetate (D, L, DL, and other C6 sugar variations), diethyl oxylate, diethyl malonate, diethyl maleate, diethyl succinate, dimethyl glutarate, diethyl glutarate, diethyl 3-hydroxy glutarate, ethyl gluconate (D, L, DL, and other C6 sugar variations), diethyl carbonate, ethylene carbonate, methyl acetoacetate, ethyl acetoacetate, butyl acetoacetate, methyl lactate, (D, L, or DL), dthyl lactate, (D, L, or DL), butyl lactate (D, L, or DL), methyl glycolate, ethyl glycolate, butyl glycolate, lactide (DD), lactide (LL), lactide (DL), glycolide, etc.

Suitable biodegradable polymeric excipients may include polylactide, polylactide-co-glycolide, polycaprolactone, etc.

Other suitable polymeric excipients include, but are not limited to, block copolymers including styrenic block copolymers such as polystyrene-polyisobutylene-polystyrene triblock copolymer (SIBS), hydrogels such as polyethylene oxide, silicone rubber and/or any other suitable polymer material.

In place of, or in addition to one or more therapeutic agents, the device of the invention may be provided with or more lubricious coatings. Examples of lubricious materials include HDPE (High Density Polyethylene) or PTFE (Polytetrafluoroethylene), or a copolymer of tetrafluoroethylene with perfluoroalkyl vinyl ether (PFA) (more specifically, perfluoropropyl vinyl ether or perfluoromethyl vinyl ether), or the like. Other suitable lubricious polymers may include silicone and the like, hydrophilic polymers such as polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Pat. Nos. 8,048,060, 7,544,381, 7,914,809, 6,673,053, and 5,509,899.

These lists are intended for illustrative purposes only, and not as a limitation on the scope of the present disclosure

According to embodiments, the invention may also relate to tubular structures having patterns configured to form any of the occlusion devices disclosed above.

The tubular structure may be microblasted and/or electropolished in order to enhance surface characteristics, long-term performance and/or biocompatibility.

Further, the present invention relates to a method of manufacturing an occlusion device for an atrial appendage. The method comprises the steps of (a) cutting a tubular body having proximal and distal ends to provide a tubular structure having struts, at least some of the struts at the distal end having loose distal strut ends; (b) expanding at least part of the tubular structure; (c) bending at least some of the loose distal strut ends towards the inside of said tubular structure such that the loose distal strut ends point in a direction towards the proximal end of the tubular structure; and (d) connecting at least some of the loose distal strut ends to each other.

The method may further comprise the step of connecting the struts at their proximal strut ends to each other so as to form a cage-like structure. In this context, the cuts may not extend to the proximal end of the tubular body, thereby connecting the struts at the proximal end by a proximal collar that is formed integrally therewith.

The distal strut ends may account for approximately 20% to 65%, preferably approximately 30% to 55%, more preferably approximately 40% to 50%, and most preferably approximately 45% of the length of the tubular structure. The tubular structure may comprise at least 6, at least 10, at least 12, or 18 distal strut ends. The struts forming the distal strut ends may differ in wall thickness and/or strut width along their entire length or a section thereof when compared to the other struts forming the tubular structure.

According to the invention, laser cutting or other suitable machining processes may be used to cut the tubular body. The tubular structure may also be provided by rolling and welding an etched and/or cut sheet of material.

The tubular structure may be heat treated and shaped over a mandrel in order to expand it and/or in order to provide the struts with a desired geometrical shape. According to an embodiment of the inventive method, a forming tool may be used for this purpose.

In some embodiments, the step of bending at least some of the distal strut ends may comprise inserting the distal struts ends into a tube located within the tubular structure. The tube may be inserted through the proximal end of the cut and expanded tubular structure and cut to length, if necessary. In this case, the step of connecting at least some of the distal strut ends to each other may comprise crimping and/or welding the tube to the loose ends, a type of connection which might be preferable from a manufacturing point of view. Additionally or alternatively, the distal strut ends may be connected to each other by welding, soldering and/or by use of adhesive.

Also, the method may comprise the step of further bending at least some of the bent struts such that the distal strut ends extend to the outside of the tubular structure and form anchor, e.g., in the form of tissue-engaging hooks or barbs.

Further according to the inventive method, at least some of the loose distal strut ends may be connected to the struts at the proximal end. In particular, at least some of the distal strut ends may be connected to the proximal collar.

The method according to the invention may further comprise the step of bending at least some of the proximal strut ends towards the inside the tubular structure such that the proximal strut ends point in a direction towards the distal end of the tubular structure. At least some of these struts may be connected to each other, for example, inside the tubular structure.

The method may further comprise the step of bending at least some of the struts such that the ends extend to the outside of the tubular structure so as to provide an anchor.

As will be appreciated by those skilled in the art, the sequence of some of the steps described above may be changed in embodiments of the invention. It is noted that the embodiments described above may be combined in any technically feasible manner and that their respective features may be provided in conjunction.

The device of the present invention may be implanted by any of the techniques known in the art, for example by the standard transseptal technique. A detailed description of methods that may be used with the device of the invention is provided, for example, in WO 03/063732.

The disclosure also may relate to methods for implanting any of the devices described above. Furthermore, the disclosure relates to a kit for implanting any of these devices, the kit comprising a device according to the invention and a corresponding implantation apparatus (e.g., an apparatus disclosed in WO 03/063732).

In view of the description provided above, it is clear that the invention provides improved devices, structures and methods meeting performance requirements and manufacturing needs.

While the invention has been illustrated and described in detail in the drawings and the foregoing description, such illustration and description are to be considered illustrative or exemplary and not restrictive. It will be understood that changes and modifications may be made by those of ordinary skill within the scope of the following claims.

## Claims

1. An occlusion device (10) for an atrial appendage, the device (10) having proximal and distal ends (12, 14) and a central axis (L) and comprising a cage-like structure (16) formed of struts (18; 218), the struts (18; 218) having proximal strut ends (20; 220) and distal strut ends (22; 122; 222), wherein at the proximal end (12) of the device (10) the struts (18; 218) extend towards the central axis (L) and are connected to each other at their proximal strut ends (20; 220), and wherein at the distal end (14) of the device (10), at least some of the struts (18; 218) extend toward the central axis (L) and are connected to each other at their distal strut ends (22; 122; 222) by a tube (32; 332) into which the distal strut ends (22; 122; 222) are inserted and/or by a collar (132) comprising several openings for receiving the distal strut ends (22; 122; 222), and wherein the device (10) further comprises a threaded insert (34) at the proximal end (12),
wherein the struts (18; 218) are connected to each other such that the distal strut ends (22; 122; 222) are located proximal to the distal most part of the device (10),
wherein the distal strut ends (22; 122; 222) in part or completely differ in wall thickness and/or a strut width from the other struts (18; 218) of the cage-like structure (16).

2. The occlusion device of claim 1, wherein the struts (18; 218) have a substantially polygonal cross section, or wherein the struts (18; 218) form a plurality of closed polygonal cells having vertices and wherein the struts (18; 218) merge into each other at said vertices.

3. The occlusion device of claim 1, wherein the distal end (14) of the device (10) is atraumatic and comprises inwardly bent struts (18; 218).

4. The occlusion device of claim 3, wherein at least some of the ends of the bent struts (18; 218) point in a direction towards the proximal end of the cage-like structure (16), or wherein at least some of the struts (18; 218) are bent such that their distal strut ends (22; 122; 222) extend substantially parallel to the central axis (L).

5. The occlusion device of claim 1, wherein at least some of the distal strut ends (22; 122; 222) provide an anchor (128).

6. The occlusion device of claim 5, wherein at least some of the struts (18; 218) providing the anchor (128) extend through the cage-like structure (16).

7. The occlusion device of claim 1, wherein at least some of the distal strut ends (22; 122; 222) are connected to proximal strut ends (20; 220).

8. The occlusion device of claim 1, wherein the proximal strut ends (20; 220) are connected to each other outside of or within the cage-like structure (16).

9. The occlusion device of claim 1, wherein the proximal strut ends (20; 220) are connected to each other by a proximal collar (30; 230) formed integrally therewith.

10. The occlusion device of claim 1, wherein the tube (32; 332) is crimped on and/or welded to the distal strut ends (22; 122; 222).

11. The occlusion device of claim 1, wherein the cage-like structure (16) is formed of a single cut structure, or wherein the cage-like structure (16) is cut from a unitary tubular body.

12. The occlusion device of claim 1, further comprising a filter (40).

13. The occlusion device of any of the preceding claims, wherein the threaded insert (34) is configured for attaching the occlusion device (10) to a tether (50) or shaft.

14. A method of manufacturing an occlusion device (10) for an atrial appendage of any one of the preceding claims, comprising the steps of:
a) cutting a tubular body having proximal and distal ends to provide a tubular structure having struts (18; 218), at least some of the struts (18; 218) at the distal end having loose distal strut ends (22; 122; 222);
b) expanding at least part of the tubular structure;
c) bending at least some of the loose distal strut ends (22; 122; 222) towards the inside of said tubular structure such that the loose distal strut ends (22; 122; 222) point in a direction towards the proximal end (12) of the tubular structure; and
d) connecting at least some of the loose distal strut ends (22; 122; 222) to each other.

## Patentansprüche

1. Okklusionsvorrichtung (10) für ein Herzohr, wobei die Vorrichtung (10) ein proximales (12) und ein distales Ende (14) und eine Mittelachse (L) aufweist und eine käfigförmige Struktur (16) aufweist, die aus Streben (18; 218) ausgebildet ist, wobei die Streben (18; 218) proximale Strebenenden (20; 220) und distale Strebenenden (22; 122; 222) aufweisen, wobei sich am proximalen Ende (12) der Vorrichtung (10) die Streben (18; 218) zur Mittelachse (L) erstrecken und an ihren proximalen Strebenenden (20; 220) miteinander verbunden sind, und wobei sich am distalen Ende (14) der Vorrichtung (10) mindestens einige der Streben (18; 218) zur Mittelachse (L) erstrecken und an ihren distalen Strebenenden (22; 122; 222) durch ein Rohr (32; 332), in das die distalen Strebenenden (22; 122; 222) eingesetzt sind, und/oder durch einen Bund (132), der mehrere Öffnungen zum Aufnehmen der distalen Strebenenden (22; 122; 222) aufweist, miteinander verbunden sind, und wobei die Vorrichtung (10) ferner einen Gewindeeinsatz (34) am proximalen Ende (12) aufweist,
wobei die Streben (18; 218) so miteinander verbunden sind, dass die distalen Strebenenden (22; 122; 222) proximal zum am weitesten distal gelegenen Teil der Vorrichtung (10) angeordnet sind,
wobei sich die distalen Strebenenden (22; 122; 222) teilweise oder vollständig in der Wanddicke und/oder eine Strebenbreite von den anderen Streben (18; 218) der käfigförmigen Struktur (16) unterscheiden.

2. Okklusionsvorrichtung nach Anspruch 1, wobei die Streben (18; 218) einen im Wesentlichen polygonalen Querschnitt aufweisen, oder wobei die Streben (18; 218) mehrere geschlossene polygonale Zellen bilden, die Vertices aufweisen, und wobei sich die Streben (18; 218) an den Vertices miteinander vereinigen.

3. Okklusionsvorrichtung nach Anspruch 1, wobei das distale Ende (14) der Vorrichtung (10) atraumatisch ist und nach innen gebogene Streben (18; 218) aufweist.

4. Okklusionsvorrichtung nach Anspruch 3, wobei mindestens einige der Enden der gebogenen Streben (18; 218) in Richtung des proximalen Endes der käfigförmigen Struktur (16) weisen oder wobei mindestens einige der Streben (18; 218) so gebogen sind, dass sich ihre distalen Strebenenden (22; 122; 222) im Wesentlichen parallel zur Mittelachse (L) erstrecken.

5. Okklusionsvorrichtung nach Anspruch 1, wobei mindestens einige der distalen Strebenenden (22; 122; 222) einen Anker (128) bereitstellen.

6. Okklusionsvorrichtung nach Anspruch 5, wobei sich mindestens einige der Streben (18; 218), die den Anker (128) bereitstellen, durch die käfigförmige Struktur (16) erstrecken.

7. Okklusionsvorrichtung nach Anspruch 1, wobei mindestens einige der distalen Strebenenden (22; 122; 222) mit proximalen Strebenenden (20; 220) verbunden sind.

8. Okklusionsvorrichtung nach Anspruch 1, wobei die proximalen Strebenenden (20; 220) außerhalb oder innerhalb der käfigförmigen Struktur (16) miteinander verbunden sind.

9. Okklusionsvorrichtung nach Anspruch 1, wobei die proximalen Strebenenden (20; 220) durch einen proximalen Bund (30; 230) miteinander verbunden sind, der einstückig mit diesen ausgebildet ist.

10. Okklusionsvorrichtung nach Anspruch 1, wobei das Rohr (32; 332) an die distalen Strebenenden (22; 122; 222) gepresst und/oder geschweißt ist.

11. Okklusionsvorrichtung nach Anspruch 1, wobei die käfigförmige Struktur (16) aus einer einzigen geschnittenen Struktur ausgebildet ist oder wobei die käfigförmige Struktur (16) aus einem einstückigen rohrförmigen Körper geschnitten ist.

12. Okklusionsvorrichtung nach Anspruch 1, die ferner einen Filter (40) aufweist.

13. Okklusionsvorrichtung nach einem der vorhergehenden Ansprüche, wobei der Gewindeeinsatz (34) zum Befestigen der Okklusionsvorrichtung (10) an einer Leine (50) oder einen Schaft konfiguriert ist.

14. Verfahren zum Herstellen einer Okklusionsvorrichtung (10) für ein Herzohr nach einem der vorhergehenden Ansprüche, das die Schritte aufweist:
a) Schneiden eines rohrförmigen Körpers, der ein proximales und ein distales Ende aufweist, um eine rohrförmige Struktur bereitzustellen, die Streben (18; 218) aufweist, wobei mindestens einige der Streben (18; 218) am distalen Ende lose distale Strebenenden (22; 122; 222) aufweisen;
b) Expandieren mindestens eines Teils der rohrförmigen Struktur;
c) Biegen von mindestens einigen der losen distalen Strebenenden (22; 122; 222) zum Inneren der rohrförmigen Struktur, so dass die losen distalen Strebenenden (22; 122; 222) in Richtung zum proximalen Ende (12) der rohrförmigen Struktur weisen; und
d) Verbinden von mindestens einigen der losen distalen Strebenenden (22; 122; 222) miteinander.

## Revendications

1. Dispositif d'occlusion (10) pour un appendice auriculaire, le dispositif (10) ayant des extrémités proximale et distale (12, 14) et un axe central (L) et comprenant une structure en forme de cage (16) formée avec des entretoises (18 ; 218), les entretoises (18 ; 218) ayant des extrémités d'entretoise proximales (20 ; 220) et des extrémités d'entretoise distales (22 ; 122 ; 222), dans lequel, au niveau de l'extrémité proximale (12) du dispositif (10), les entretoises (18 ; 218) s'étendent vers l'axe central (L) et sont raccordées entre elles au niveau de leurs extrémités d'entretoise proximales (20 ; 220), et dans lequel au niveau de l'extrémité distale (14) du dispositif (10), au moins certaines des entretoises (18 ; 218) s'étendent vers l'axe central (L) et sont raccordées entre elles au niveau de leurs extrémités d'entretoise distales (22 ; 122 ; 222) par un tube (32 ; 332) dans lequel les extrémités d'entretoise distales (22 ; 122 ; 222) sont insérées et/ou par un collier (132) comprenant plusieurs ouvertures pour recevoir les extrémités d'entretoise distales (22 ; 122 ; 222), et dans lequel le dispositif (10) comprend de plus un insert fileté (34) au niveau de l'extrémité proximale (12),
dans lequel les entretoises (18 ; 218) sont raccordées entre elles de sorte que les extrémités d'entretoise distales (22 ; 122 ; 222) sont disposées à proximité de la partie la plus distale du dispositif (10),
dans lequel les extrémités d'entretoise distales (22 ; 122 ; 222) diffèrent en partie ou complètement du point de vue de l'épaisseur de paroi et/ou de la largeur d'entretoise des autres entretoises (18 ; 218) de la structure en forme de cage (16).

2. Dispositif d'occlusion selon la revendication 1, dans lequel les entretoises (18 ; 218) présentent une section transversale sensiblement polygonale, ou dans lequel les entretoises (18 ; 218) forment une pluralité de cellules polygonales fermées ayant des sommets et dans lequel les entretoises (18 ; 218) fusionnent entre elles au niveau desdits sommets.

3. Dispositif d'occlusion selon la revendication 1, dans lequel l'extrémité distale (14) du dispositif (10) est atraumatique et comprend des entretoises pliées vers l'intérieur (18 ; 218).

4. Dispositif d'occlusion selon la revendication 3, dans lequel au moins certaines des extrémités des entretoises pliées (18 ; 218) pointent dans une direction vers l'extrémité proximale de la structure en forme de cage (16), ou dans lequel au moins certaines des entretoises (18 ; 218) sont pliées de sorte que leurs extrémités d'entretoise distales (22 ; 122 ; 222) s'étendent de manière sensiblement parallèle jusqu'à l'axe central (L).

5. Dispositif d'occlusion selon la revendication 1, dans lequel au moins certaines des extrémités d'entretoise distales (22 ; 122 ; 222) fournissent un ancrage (128).

6. Dispositif d'occlusion selon la revendication 5, dans lequel au moins certaines des entretoises (18 ; 218) fournissant l'ancrage (128) s'étendent à travers la structure en forme de cage (16).

7. Dispositif d'occlusion selon la revendication 1, dans lequel au moins certaines des extrémités d'entretoise distales (22 ; 122 ; 222) sont raccordées aux extrémités d'entretoise proximales (20 ; 220).

8. Dispositif d'occlusion selon la revendication 1, dans lequel les extrémités d'entretoise proximales (20 ; 220) sont raccordées entre elles à l'extérieur de ou à l'intérieur de la structure en forme de cage (16).

9. Dispositif d'occlusion selon la revendication 1, dans lequel les extrémités d'entretoise proximales (20 ; 220) sont raccordées entre elles par un collier proximal (30 ; 230) formé de manière solidaire avec ces dernières.

10. Dispositif d'occlusion selon la revendication 1, dans lequel le tube (32 ; 332) est serti sur et/ou soudé sur les extrémités d'entretoise distales (22 ; 122 ; 222).

11. Dispositif d'occlusion selon la revendication 1, dans lequel la structure en forme de cage (16) est formée avec une structure découpée unique, ou dans laquelle la structure en forme de cage (16) est découpée à partir d'un corps tubulaire unitaire.

12. Dispositif d'occlusion selon la revendication 1, comprenant de plus un filtre (40).

13. Dispositif d'occlusion selon l'une quelconque des revendications précédentes, dans lequel l'insert fileté (34) est configuré pour fixer le dispositif d'occlusion (10) à une attache (50) ou un arbre.

14. Procédé pour fabriquer un dispositif d'occlusion (10) pour un appendice auriculaire selon l'une quelconque des revendications précédentes, comprenant les étapes consistant à :
a) couper un corps tubulaire ayant des extrémités proximale et distale pour fournir une structure tubulaire ayant des entretoises (18 ; 218), au moins certaines des entretoises (18 ; 218) au niveau de l'extrémité distale ayant des extrémités d'entretoise distales lâches (22 ; 122 ; 222) ;
b) faire subir une extension à au moins une partie de la structure tubulaire ;
c) plier au moins certaines des extrémités d'entretoise distales (22 ; 122 ; 222) lâches vers l'intérieur de ladite structure tubulaire de sorte que les extrémités d'entretoise distales (22 ; 122 ; 222) lâches se dirigent dans une direction vers l'extrémité proximale (12) de la structure tubulaire ; et
d) raccorder au moins certaines des extrémités d'entretoise distales (22 ; 122 ; 222) lâches entre elles.
